Europäisches Patentamt

European Patent Office

Office européen des brevets

(11) Publication number: **0 286 303 B1**

(12) **EUROPEAN PATENT SPECIFICATION**

(45) Date of publication of patent specification: **10.02.93**

(51) Int. Cl.5: **C12N 15/52**, C12N 15/75, C12P 19/40, C12P 1/04

(21) Application number: **88302827.6**

(22) Date of filing: **30.03.88**

(54) **DNA and its use.**

(30) Priority: **01.04.87 JP 82096/87**

(43) Date of publication of application:
**12.10.88 Bulletin 88/41**

(45) Publication of the grant of the patent:
**10.02.93 Bulletin 93/06**

(84) Designated Contracting States:
**AT BE CH DE ES FR GB GR IT LI LU NL SE**

(56) References cited:
**EP-A- 0 151 341**
**GB-A- 2 124 225**

**JOURNAL OF BACTERIOLOGY , vol. 107, no. 3, September 1971 (Washington, DC) B.-Z-DORFMAN "Allelic Variability in Comparative Complementation Confirming that the ade 12-Specified Protein of Yeast is Bifunctional", pp. 646-654**

**PATENT ABSTRACTS OF JAPAN, unexamined applications, field C, vol. 7, no. 281, December 15, 1983 - THE PATENT OFFICE JAPANESE GOVERNMENT, page 37 C 200**

H:J: PEPPLER; D. PERLMAN **"Microbial Technology", 2nd edition, vol. 1, 1979, Academy Press, New York - Y. NAKAO "Microbial Production of Nucleosides and Nucleotides" pages 311-354**

(73) Proprietor: **Takeda Chemical Industries, Ltd.
1-1, Doshomachi 4-chome
Chuo-ku, OSAKA(JP)**

(72) Inventor: **Miyagawa, Kenichiro
6-11, Higashitokiwadai 6-chome Toyono-cho
Toyono-gun Osaka 663-01(JP)**
Inventor: **Kanzaki, Naoyuki
17-201,8 Kuragauchi 2-chome
Ibaraki Osaka 567(JP)**
Inventor: **Sumino Yasuhiro
6-22, Higashiochiai 3-chome
Suma-ku Kobe Hyogo 654(JP)**

(74) Representative: **Lewin, John Harvey et al
Elkington and Fife Prospect House 8 Pembroke Road
Sevenoaks, Kent TN13 1XR(GB)**

Rank Xerox (UK) Business Services

**Description**

The present invention relates to a production method for inosine and guanosine, substances which are important as starting materials for the synthesis of 5′-inosinic acid and 5′-guanylic acid, to a new microorganism used for the production, and to a new DNA used for constructing said new microorganism.

For producing inosine and guanosine by fermentation, are known the methods using Bacillus strains (Japanese Published Examined Patent Application Nos. 2956/1980, 14160/1982 and 41915/1982, Japanese Published Unexamined Patent Application No. 42895/1984) or Brevibacterium strains [Japanese Published Examined Patent Application No. 5075/1976; Agricultural Biological Chemistry, 42, 399 (1978)] which have an adenine requirement or which have not only an adenine requirement but also various drug resistances.

As a result of many research works which have been conducted on nucleic acid fermentation, it is known that the biosynthesis of purine nucleotide related substances, such as inosine, guanosine, xanthosine, adenosine, bases therefrom (e.g. hypoxanthine) and phosphates therefrom (e.g. 5′-inosinic acid) are subjected to feedback control by adenine related substances (adenosine or phosphate therefrom such as 5′-adenylic acid) or guanine related substances (guanosine or phosphate therefrom such as 5′-guanylic acid), the end products. Specifically, the inhibition of the biosynthesis path for these purine nucleotide related substances by adenine related substances is noticeable; therefore, it is normal to use an adenine-requiring mutant lacking adenylosuccinate synthetase as well as to limit the amount of adenine related substances in the medium, to thereby maintain the amount of adenine related substances in the culture broth at low level (Y. Nakao, Microbial Technology, vol. 1, pp. 311-354, edited by H. J. Peppler and D. Perlman, Academic Press, New York). For the derivation of adenine requirement, there has conventionally been employed the method in which an adenine-requiring auxotroph is selected by the replica plate method etc. following mutagenic treatment such as ultraviolet irradiation and nitrosoguanidine (NTG) treatment.

However, the adenine-requiring mutant thus obtained have some drawbacks; for example, growth is poor because the mutation of the relevant enzyme gene is often accompanied by mutations on other sites on the chromosome, and back mutation during the fermentation reduces the efficient and stable productivity of inosine and/or guanosine. Specifically in the case of fermentative production on an industrial scale, the frequency of back mutation increases due to the increase in the frequency of cell division, and, as a result, the drawbacks become more serious.

Taking note of these circumstances, the present inventors made investigations of how to obtain an adenylosuccinate synthetase deficient strain; as a result, the inventors succeeded in constructing a new microorganism whose chromosomal DNA was converted by recombinant DNA techniques as follows: an appropriate DNA fragment except for DNA encoding adenylosuccinate synthetase was inserted into the adenylosuccinate synthetase gene region on the chromosome of the said microorganism. The inventors found that very stable production can be repeatedly performed because said new microorganism derivated by said technology from a microorganism selected from Bacillus strain as the recipient accumulates a large amount of inosine and/or guanosine, and back mutation of adenylosuccinate synthetase requirement during fermentation is not detected. Based on this finding, the present invention has been developed.

Accordingly, the present invention provides:

(1) a DNA comprising an adenylosuccinate synthetase gene region with a DNA fragment inserted to render said gene inactivated,

(2) the DNA as defined in above term (1) in which said DNA is a gene other than an adenylosuccinate synthetase gene,

(3) the DNA as defined in above term (2) in which said other gene is a selection marker gene,

(4) a vector with a DNA as defined in the above term (1), (2) or (3) inserted therein,

(5) a Bacillus strain which is a transformant resulting from transformation with the DNA as defined in the above term (3) or with the vector as claimed in the above term (4), which lacks adenylosuccinate synthetase and which is capable of producing inosine and/or guanosine, and

(6) a method of producing inosine and/or guanosine, which comprises cultivating in a medium a Bacillus strain which is a transformant resulting from transformation with a DNA comprising an adenylosuccinate synthetase gene region with a DNA fragment inserted to render said gene inactivated or with a vector DNA which lacks adenylosuccinate synthetase and which is capable of producing inosine and/or guanosine allowing said strain to produce and accumulate inosine and/or guanosine, and harvesting inosine and/or guanosine so produced in the culture medium.

It is desirable that the DNA containing an adenylosuccinate synthetase gene region used for this invention be a DNA containing both the entire DNA segment encoding said enzyme gene and some flanking regions directly upstream and downstream the DNA segment, and a DNA which contains a part of said enzyme gene or which contains both a part of said enzyme gene and flanking regions thereof may be used

2

for present invention. Such DNA is easily isolated from microbial chromosomal DNA by recombinant DNA techniques. For host-vector systems for this purpose, normally used EK systems are appropriately used. That is, mutants derived from Escherichia coli K-12, for example Escherichia coli C600 [T. Maniatis et al.: Molecular Cloning-A Laboratory Manual, Cold Spring Harbor Laboratory, University of Tokyo Press, 1982, p. 504], Escherichia coli JA221 (IFO 14359, ATCC 33875), Escherichia coli MM 294 (ATCC 33625) [Proceedings of Natural Academy of Science, USA, 73, 4174 (1976)] and derivatives thereof, are appropriately used as hosts; pBR 322, pACYC 184, pACYC 177, pUC 18, pUC 19, pHSG 396, pHSG 298, etc. are appropriately used as vectors.

In addition, there may also be used host-vector systems which are combinations of a phage vector such as Charon 4A ("Molecular Cloning", p. 31), EMBL 3 or EMBL 4 (Journal of Molecular Biology, 170, 827) and a host such as Escherichia coli DP 50supF ("Molecular Cloning", p. 504), Escherichia coli NM 538 or NM 539 (Journal of Molecular Biology, 170, 827), Escherichia coli LE 392 (Journal of Biological Chemistry, 218, 97), or the like. It is also possible to use host-vector systems of a Bacillus strain, for example, Bacillus subtilis MI 114 (Gene, 24, 255) or a mutant derived therefrom, as the host and a plasmid capable of autonomous replication in Bacillus strain, for example, pUB 110, pC 194, pE 194, pS 194, pSA 2100, or pHY 300 PLK, as the vector.

In general, there is no special limitation to the origin of the adenylosuccinate synthetase gene, so any microorganism can be used as a donor, as long as the base sequence of its adenylosuccinate synthetase gene has high homology with the base sequence of the adenylosuccinate synthetase gene on the chromosome of the Bacillus strain used as the recipient described below. Specifically, the use of a Bacillus strain is preferred, also from the point of view of easy handling, because what is called self cloning is achieved, and it also gives expectation that the obtained transformant will be stable. Furthermore, said donor does not always need to be capable of producing inosine, guanosine, xanthosine or purine bases thereof, but it is preferable that the donor has no adenylosuccinate synthetase deficiency. As examples of such DNA donors, mention may be made of Bacillus strains such as Bacillus subtilis, Bacillus pumilus and Bacillus licheniformis. More specifically, the following strains may be mentioned as examples thereof.

Bacillus subtilis NO. 168 (BGSC 1A1)

Bacillus subtilis No. 115 (IFO 14187, FERM BP-1327)

Bacillus subtilis MI 114 (Gene, 24, 255)

Bacillus pumilus ATCC 7061

Bacillus licheniformis ATCC 14580

| | |
|---|---|
| BGSC : | Bacillus Genetic Stock Center |
| IFO : | Institute for Fermentation, Osaka |
| FERM BP: | Accession number based on the Budapest Treaty at Fermentation Research Institute (FRI), Agency of Industrial Science and Technology, Ministry of International Trade and Industry, 1-3, Higashi 1-chome, Yatabemachi, Tsukuba-gun, Ibaragi-ken, Japan |
| ATCC: | American Type Culture Collection |

For preparing chromosomal DNA from the donor, a known method, for example the method using phenol to extract chromosomal DNA (H, Saito and K. Miura: Biochem. Biophy. Acta. 72,619), is used. The chromosomal DNA thus obtained is digested with appropriately chosen restriction enzymes, and is ligated with a vector by means of DNA ligase. This ligation mixture is used to transform a host bacterium which is deficient in adenylosuccinate synthetase, and transformant in which said deficiency has been complemented is selected. The transformation of the adenylosuccinate synthetase-deficient host can be conducted in accordance with a known method, for example, the method of S. N. Cohen et al. (Proceedings of National Academy of Science, USA, 69, 2110) when the host is Escherichia coli, or in accordance with a known method, for example, the method of S. Chang and S. N. Cohen (Molec. Gen. Genet., 168, 115) when the host is a Bacillus strain.

Whether the adenylosuccinate synthetase deficiency has been complemented in the transformant thus obtained can easily be judged on the basis of growability of said transformant in adenine-free medium- -medium supplemented with nutrients other than adenine as required--.

For obtaining the DNA containing said gene in a large amount from the transformant which has had the adenylosuccinate synthetase deficiency complemented, the procedure can be performed in accordance with the method described in above "Molecular Cloning", pp. 86-93.

This recombinant DNA technique can be conducted in accordance with the method described in Japanese Published Unexamined Patent Application No. 156388/1985 or European Patent Publication No. 0151341.

DNA containing said gene can also be obtained by selecting from a gene library of the donor a recombinant which complements the adenine requirement of said enzyme-deficient mutant of a Bacillus strain having a chromosome which is highly homologous with the base sequence of the adenylosuccinate synthetase gene of a Bacillus strain used as the recipient described below.

A gene library can be prepared, for example in accordance with the method of G. Rapoport et al. (Molec. Gen. Genet. 176, 239) or the method of E. Ferrari et al. (J. Bacteriol. 146, 430) in the manner summarized below. The chromosomal DNA extracted from the donor is digested with appropriately selected restriction enzymes and ligated with a vector by means of DNA ligase. The ligation mixture is used to obtain a transformant. From said transformant the recombinant plasmid is extracted, for example in accordance with the method described in "Molecular Cloning":, p. 368, and which is used as a gene library. Then, the abovementioned recombinant plasmid is given to a competent cell prepared from an adenylosuccinate synthetasedeficient strain of the genus Bacillus, and a recombinant plasmid capable of complementing adenylosuccinate synthetase deficiency of said strain, whereby a DNA containing the adenylosuccinate synthetase gene for the present invention can be obtained.

For constructing and isolating the new DNA of this invention from the DNA containing adenylosuccinate synthetase gene thus obtained, recombinant DNA technology is easily and conveniently used; for example, a host-vector system of Escherichia coli is appropriately used.

The new DNA of this invention can be constructed and isolated from a plasmid containing the adenylosuccinate synthetase gene in the following procedure.

The plasmid containing the adnylosuccinate synthetase gene is digested with a restriction enzyme by a conventional method. Separately, the DNA fragment to be inserted is digested with a restriction enzyme by a conventional method, and the cleaved fragment, after being separated for example by agarose gel electrophoresis, is extracted and isolated if necessary. The two fragments are mixed together, and, when the cohesive ends are identical with each other, both fragments are directly ligated together by means of T4 DNA ligase, or, when the cohesive ends are not identical with each other, both fragments are ligated together by means of T4 DNA ligase after converting the cohesive ends to blunt ends by means of T4 DNA polymerase, whereby the desired DNA is obtained.

For digesting the plasmid containing the adenylosuccinate synthetase gene, any restriction enzyme can be used, as long as it finds cleavage sites on the gene DNA, and especially those which find less cleavage sites on the gene and which do not cleave any other portions of the plasmid are preferably chosen. There may be used as appropriate, for example, Afl II, Apa I, Axy I, Bam HI, Bcl I, Bgl II, Bst EII, Cla I, Eco RI, Eco 81I, Hpa I, Hin dIII, Kpn I, Mlu I, Nco I, Pst I, Sac I, Sac II, Sal I, Sma I, Sph I, Spl I, Ssp I, Stu I, Xba I, and Xho I.

For the DNA fragment to be inserted in the adenylosuccinate synthetase gene region in this invention, any DNA fragment can be used irrespective of origin, including DNA fragments from prokaryotes and eukaryotes, as long as it inactivates said gene. From the viewpoint of practical application, it is normally advantageous to use a DNA fragment containing a selection marker gene so that the selection of transformants is facilitated as well as adnylosuccinate synthetase is deficient.

For such a selection marker gene, any gene can be used, as long as it expresses itself in a Bacillus strain, but it is preferable that the marker gene express itself in an Escherichia coli host. In this case, the expression in a Bacillus strain and the expression in an Escherichia coli host may be of what is called readthrough; therefore, the DNA fragment to be inserted does not always need to have a promoter which expresses itself in said host.

As examples of such marker genes, mention may be made of drug resistance genes, amino acid biosynthesis genes, nucleic acid biosynthesis genes, and vitamin biosynthesis genes. Drug resistance genes include, for example, Bacillus pumilus chloramphenicol acetyltransferase gene [D.M. Williams et al.: J. Bacteriol., 146, 1162 (1981)], plasmid pC 194 chloramphenicol acetyltransferase gene [S. Horinouchi and B. Weisblum: J. Bacteriol., 150, 815 (1982)] and plasmid pUB 110 kanamycin nucleotidyltransferase gene [M. Matsumura et al.: J. Bacteriol., 160, 413 (1984)] Amino acid biosynthesis genes include Bacillus subtilis leucine biosynthesis gene [T. Tanaka and K. Sakaguchi: Molec. Gen. Genet., 165, 269 (1978)] and tryptophan biosynthesis gene [K. Yoshimura et al.: J. Bacteriol., 159, 905 (1984)]; nucleic acid biosynthesis genes include Escherichia coli thymidine synthetase gene [Rubin et al.: Gene, 10, 227 (1980)]; and vitamin biosynthesis genes include mouse dihydrofolate reductase gene [D. M. Williams et al.: Gene, 16, 199 (1981)].

For example, the selection of transformant carrying an inserted inactivated adenylosuccinate synthetase gene can easily be performed by a method such as the method in which the relevant antibiotic is added to the selection medium when a DNA containing a drug resistant gene is used, or by complementation of auxotrophy when a biosynthesis gene is used as the marker.

A selection marker gene DNA fragment can be constructed as follows:

For isolating a selection marker gene DNA, a recombinant DNA carrying the gene is digested with an appropriately chosen restriction enzyme and fractionated by agarose gel electrophoresis, and the desired DNA is then isolated from the agarose gel fragment containing the gene by a known method, for example, the method described in "Molecular Cloning", pp. 164-166. For the restriction enzyme used for this purpose, those which find no restriction sites on said gene and which provide as small a fragment containing said gene as possible are preferably chosen. It is also possible to use appropriate two restriction enzymes in combination if necessary. When the both ends of the selection marker gene DNA fragment thus obtained are not identical with the cohesive ends of the restriction sites of the adenylosuccinate synthetase gene to be inserted, ligation becomes possible by using T4 DNA polymerase to convert said cohesive ends to blunt ends.

For obtaining the new DNA of the present invention in a large amount, the host bacterium is transformed using the above-mentioned ligation mixture; a transformant grown in the selection medium corresponding to the inserted selection marker is obtained; and this transformant is subjected to the procedure in accordance with a known method such as the above-mentioned "Molecular Cloning", pp. 86-93.

For isolating the adenylosuccinate synthetase gene region having the marker inserted therein from the plasmid thus obtained, said plasmid is digested with restriction enzymes by a conventional method and separated by agarose gel electrophoresis, after which it can easily be extracted and isolated, for example, by means of an electroeluter.

Next, an explanation is given of the method of constructing a new microorganism by transforming a Bacillus strain by means of the new DNA thus obtained.

As the recipient for this transformation, any bacterium can be used, as long as it possesses the characteristic in which the bacterium uptakes the linear DNA given outside the cell and changes its character as a result of the recombination at the portion which is highly homologous with the base sequence of said DNA on the chromosome, namely what is called the capability of transformation; the recipient does not need to be identical with the adenylosuccinate synthetase gene donor, as long as the base sequence of its chromosome DNA is a highly homologous to the base sequence of the adenylosuccinate synthetase gene contained in the linear DNA. For the purpose of obtaining transformants which are highly capable of producing inosine and/or guanosine, Bacillus strains, specifically Bacillus subtilis, Bacillus pumilus, Bacillus licheniformis, and the like are preferred from the view point of the fact that said bacteria are known to accumulate at least one of the purine nucleotide related substances, for example, inosine, guanosine and xanthosine, capable of transformation, free from pathogenicity, and have long been safely used on industrial scale.

In addition, for example, when these bacteria, after being provided with one or more than one of the known characteristics which are regarded as advantageous for the accumulation of purine nucleotide related substances, for example, purine analogue resistances such as 8-azaguanine resistance, nucleoside phosphorylase deficiency, 5′-guanylate reductase deficiency and folate antagonist resistance, are used as recipients, there are often obtained results which are more favorable for improving inosine and/or guanosine productivity.

For the purpose of advantageously producing inosine according to this invention, it is desirable to use as the recipient a Bacillus strain which is a transformant resulting from transformation with a DNA comprising a 5′-inosinic acid dehydrogenase (IMP dehydrogenase) gene region which has had a DNA fragment inactivating the gene inserted therein or with a vector carrying said DNA inserted therein, which lacks IMP dehydrogenase and which is capable of producing inosine (disclosed in Japanese Patent Applicatin No. 311588/1986 and the U.S. Patent Application Serial No. 136,367). As an example of the recipient, mention may be made of Bacillus subtilis BM 1041 (IFO 14560, FERM BP-1243).

In addition, when conforming to the method of this invention, it is possible to use a strain possessing no adenylosuccinate synthetase deficiency as the recipient to construct the new microorganism of this invention, and it is also possible to use an adenylosuccinate synthetase deficient strain which has become capable of producing purine nucleotid related substances as a result of breeding by a conventional method as the recipient to obtain a new stable microorganism with improved capability of purine nucleotide related substances production. As examples of recipients belonging to the genus Bacillus, mention may by made of the following:

Bacillus subtilis No. 115 (IFO 14187, FERM BP-1327)
Bacillus subtilis No. 168 (BGSC No. 1A1)
Bacillus subtilis MI 114 (Gene 24.255)
Bacillus subtilis BM 1022 (IFO 14580, FERM BP-1324)
Bacillus subtilis BM 1032 (IFO 14559, FERM BP-1242)
Bacillus subtilis 1A3 (BGSC No. 1A3)

Bacillus subtilis NA-6011 (IFO 14189, FERM BP-291)
Bacillus subtilis NA-6012 (IFO 14190, FERM BP-292)
Bacillus subtilis NA-6128 (IFO 14373, FERM BP-617)
Bacillus subtilis NA-7821 (IFO 14368, FERM BP-618)
Bacillus subtilis ATCC 19221
Bacillus subtilis ATCC 14662
Bacillus pumilus ATCC 7061
Bacillus pumilus (FERM P-2116)
Bacillus pumilus (FERM P-4832)
Bacillus pumilus (FERM P-4829)
Bacillus licheniformis ATCC 14580
Bacillus licheniformis IFO 12485

When the new DNA of this invention is used to transform a Bacillus strain, the DNA can be used in the form of an insert in the plasmid; however, in general, it is preferable to use a linear form DNA or the DNA of this invention out of the plasmid.

Transformation of a Bacillus strain using a linear DNA fragment can be performed by a known method [C. Anagnostopouls and J. Spizizen: J. Bacteriol., 81, 741 (1961)], and the transformant can be picked up from the colony grown on the selection medium by means of a selection marker. For example, when a drug resistance gene is inserted as the selection marker, the transformant can be picked up from the selection medium containing the relevant drug. For judging whether or not the transformant thus obtained has become an adenylosuccinate synthetase deficient strain as desired in this invention, its growth is examined on each of a minimum medium containing substances essential to the growth of the recipient and a supplemented medium prepared by adding adenine to said minimum medium.

When the recipient used for the transformation lacks adenylosuccinate synthetase, the change on the adenylosuccinate synthetase gene cannot be judged by said method because the recipient itself does not grow in the above-mentioned medium without adenine, but it is easily possible to use Southern hybridization as described in the next paragraph to thereby judge whether the obtained transformant is the desired transformant. A microorganism of this invention is easily obtained even when the recipient used for transformation lacks adenylosuccinate synthetase. In order to repair adenylosuccinate synthetase deficiency, the recipient is transformed with a DNA containing the normal adenylosuccinate synthetase gene, which has been cloned from a donor microorganism having adenylosuccinate synthetase activity. Then, the transformant was re-transformed with the DNA of this invention.

Whether the transformant thus obtained is the desired new microorganism can be confirmed by digesting the chromosome DNA of said transformant with appropriate restriction enzymes, subjecting to agarose gel electrophoresis, denaturing with alkali, transferring to a nitrocellulose filter, separately radio-labeling a DNA fragment as used for the insertion, for example, a DNA fragment containing drug resistance gene, and performing Southern hybridization using the radiolabeled DNA fragment as the probe. That is, it can easily be judged that said transformant is the microorganism as desired in this invention, based on the fact that no fragment hybridizing to the probe is present in the cromosome DNA of the recipient while a fragment hybridizing to the probe is present in the chromosome DNA of said transformant.

It can be confirmed that said DNA fragment has been inserted on the adenylosuccinate synthetase gene, based on the fact that, when Southern hybridization is performed using the adenylosuccinate synthetase isolated from the donor as the probe, the fragment of the chromosome DNA of the transformant, hybridizing to the probe has become greater than the DNA fragment of the recipient by the length of the inserted DNA fragment.

The above-mentioned transformant which has had the marker gene inserted therein on the adenylosuccinate synthetase gene on the chromosome DNA, has become, for example, drug resistant as a result of the expression of the marker gene when the marker gene is a drug resistance gene, and has become adenylosuccinate synthetase deficient and adenine-requiring as a result of the insertion of the marker gene, but the transformant remains unchanged in the other bacteriological characteristics when compared to the recipient before transformation.

The characteristic of said transformant include that the rate of back mutation for adenine requirement is so low that revartant is not detected.

The rate of back mutation can be expressed in terms of the ratio of the number of colonies grown on adenine-free agar plate medium to the number of cells spread over said agar plate medium with adenine. However, the rate of back mutation in said transformant is extremely low; no revertant appears even when the transformant is subcultured or treated with mutagenic agent.

As examples of such transformants of this invention, mention may be made of Bacillus subtilis BM 1051

6

(IFO 14581, FERM BP-1325) and Bacillus subtilis NA-6201 (IFO 14582, FERM BP-1326), as shown under Examples below. It is of course easily possible to construct various transformants in the same manner in accordance with the method described in the present specification by choosing selection marker genes and recipients as appropriate.

For producing inosine and/or guanosine using the transformant obtained by this invention, the same method as the conventional method of cultivating bacteria which produce inosine and/or guanosine is employed.

That is, for the medium, a medium containing nutrients such as amino acid, nucleic acid and vitamins as required, as well as carbon source, nitrogen source and metal ions, is used. For the carbon source, glucose, sucrose, maltose, starch, saccharified starch liquid, molasses, etc. are used. For the nitrogen source, inorganic nitrogen sources such as ammonium salts of sulfuric acid, nitric acid, hydrochloric acid, carbonic acid, etc., ammonia gas and aqueous ammonia, as well as organic nitrogen sources such as peptone, corn steep liquor, soybean meal, dry yeast and urea are used singly or in combination. For the other nutrients, various minerals, amino acids, vitamins, etc., essential for the growth of the bacterium are chosen as appropriate and used singly or in combination. For the adenine source, not only adenine, adenosine, adenylic acid, and nucleic acid but also microbial cells containing these substances, extracts therefrom, etc. are used.

Furthermore, the medium may have defoaming agents such as silicone oil and polyalkylene glycol and surfactants added thereto if necessary.

Cultivation is normally carried out under aerobic conditions, i.e., shaking culture, aerobic submerged culture, etc. It is preferable that the pH of the medium be in the range of from 4 to 9. When a fluctuation in pH is observed during cultivation, sulfuric acid, calcium carbonate, sodium hydroxide, ammonia gas, aqueous ammonia, etc. may be added as appropriate to readjust the pH to the preferred range. Culturing temperature is normally chosen from the range of from 20°C to 45°C as appropriate for the growth of the microorganism used and for the accumulation of inosine and/or guanosine. Cultivation is continued until the amount of inosine and/or guanosine substantially reaches the maximum, but the purpose is normally accomplished in 24 to 144 hours of cultivation.

For separating and harvesting inosine and/or guanosine from the culture, ordinary purification means which have already been publicized are used, for example, precipitation, and chromatography using ion-exchange resin or activated charcoal.

The present invention enables the efficient, industrially advantageous production of inosine and guanosine, which are the raw materials of the important seasoning substances 5′-inosinic acid and 5′-guanylic acid.

That is, a new bacterial strain which lacks adenylosuccinate synthetase and which is highly capable of producing inosine and/or guanosine can be obtained by transforming a Bacillus strain as the recipient using the DNA of this invention.

Furthermore, by this invention it is also possible to obtain a new bacterial strain with further improved productivity using an already bred adenylosuccinate synthetase-deficient Bacillus strain capable of producing any one or more of inosine, xanthosine, and guanosine as the recipient. Said producer bacterium not only has a high accumulation of inosine and guanosine, but also is unlikely to present back mutation so that it is very stable; therefore, the bacterium is very advantageous from the viewpoint of production management.

The present invention will now be illustrated in more detail by means of the following working examples, but of course this invention is never limited thereto. The reactions using the restriction enzymes (all produced by Nippon Gene, Japan) or modifying enzymes in the following examples were carried out using the reaction conditions specified by the enzyme producers unless otherwise stated.

Depositories and date of deposite of 8 microorganisms referred to in the following Examples are shown in Table below:

| Depository<br><br>Microorganism | IFO<br>(date of deposite) | FRI<br>(date of deposite) |
| --- | --- | --- |
| <u>Bacillus subtilis</u><br>BM 1051 | 14581<br>(March 12, 1987) | FERM BP-1325<br>(March 28, 1987) |
| <u>Bacillus subtilis</u><br>NA-6201 | 14582<br>(March 18, 1987) | FERM BP-1326<br>(March 28, 1987) |
| <u>Bacillus subtilis</u><br>No. 115 | 14187<br>(July 13, 1982) | FERM BP-1327<br>(March 28, 1987) |
| <u>Bacillus subtilis</u><br>BM 1032 | 14559<br>(December 22,<br>1986) | FERM BP-1242<br>(December 25, 1986) |
| <u>Escherichia coli</u><br>MM 294 (pPA350-1) | 14579<br>(March 18, 1987) | FERM BP-1323<br>(March 28, 1987) |
| <u>Escherichia coli</u><br>C 600 (pPA350) | 14578<br>(March 18, 1987) | FERM BP-1322<br>(March 28, 1987) |
| <u>Bacillus subtilis</u><br>NA-6128 | 14373<br>(September 18,<br>1984) | FERM BP-617<br>(September 25, 1984) |
| <u>Bacillus subtilis</u><br>BM 1022 | 14580<br>(March 18, 1987) | FERM BP-1324<br>(March 28, 1987) |

Example 1

i) Preparation of chromosome DNA

Bacillus subtilis No. 115 (IFO 14187, FERM BP-1327) was inoculated to C medium (5 g/ℓ glucose, 0.5 g/ℓ sodium citrate, 5 g/ℓ polypeptone, 5 g/ℓ yeast extract, 1 g/ℓ (NH₄)₂SO₄, 7 g/ℓ K₂HPO₄, 3 g/ℓ KH₂PO₄, 0.2 g/ℓ MgSO₄. 7H₂O, pH7.0) and cultured at 37°C overnight, and 7.1 mg of chromosome DNA was obtained from 1 ℓ of the culture broth by the chromosome DNA extraction method using phenol.

ii) Fractionation of chromosome DNA

The chromosome DNA obtained in the term i) was reacted with the restriction enzyme Bgl II at 37°C for 60 minutes for complete digestion. The reaction mixture was then fractionated to twenty about 500 μℓ portions by the sucrose gradient method (sucrose concentration, 10 to 40%; Beckmann ultracentrifuge roter SW41Ti, 26000 rpm, 24 hours, 20°C). The sucrose gradient method was conducted in accordance with the method described in "Molecular Cloning", pp.270-280.

Then, using an aliquot of each fraction, competent cells of Bacillus subtilis BM 1032, the adenylosuccinate synthetase deficient strain prepared by the conventional method described later, were transformed, and they were spread over M-9 CAT agar plate medium (6 g/ℓ Na₂HPO₄, 3 g/ℓ KH₂PO₄, 0.5 g/ℓ NaCl, 1 g/ℓ NH₄Cl, 1mM MgSO₄, 1 mM CaCl₂, 2 g/ℓ glucose, 2 g/ℓ casamino acid, 50 mg/ℓ tryptophan, 15 g/ℓ agar, pH 7.2); as a result, there appeared non-adenine-requiring colonies from the fraciton numbers 4,5, and

8

6. The fraction numbers 4,5, and 6 were collected and subjected to ethanol precipitation, and DNA was recovered.

The DNA precipitate was then dissolved in TE buffer, and the restriction enzyme Bam HI was allowed to act thereon at 37°C for 1 hour to thereby completely digest the DNA, and, then, the reaction mixture was subjected to agarose gel electrophoresis. The agarose gel was cut into 8 gel fragments according to mobility, and DNA was recovered from each fragment by means of a Unidirectional Electroeluter (International Biotechnologies, Inc.). Using an aliquot of the DNA, competent cells of Bacillus subtilisBM 1032 were transformed in the same manner as above, whereby a DNA fragment capable of complementing the adenine requirement of said strain was obtained from the fraction corresponding to the electrophoretic mobility of about 6.7 to 8.5 kb DNA.

iii) Insertion of chromosome DNA fragment into pBR322

After being digested with the restriction enzyme Bam HI, 3 $\mu$g of pBR322 was treated with alkaline phosphatase and subjected to phenol extraction followed by ethanol precipitation The resulting precipitate was dissolved in TE buffer and mixed with the DNA fragment of the term ii), and ligation reaction was carried out by means of T4 DNA ligase (Takara Shuzo, Japan). Using 5 $\mu\ell$ of this reaction mixture, competent cells of Escherichia coli C600 strain were transformed, whereby about 800 transformants which are resistant to ampicillin and sensitive to tetracycline were obtained. This transformation was conducted in accordance with the method of Cohen et al. Then, DNA was extracted from each of the above-mentioned Escherichia coli transformants in accordance with the method described in "Molecular Cloning", pp.368-369, and the competent cells of Bacillus subtilis BM 1032 prepared in the term ii) were transformed, whereby a transformant carrying the recombinant plasmid complementing the adenine requirement of said strain, namely Escherichia coli C600 (pPA350), was obtained.

iv) Large-scale preparation of recombinant plasmid from transformant Escherichia coli C600 (pPA350) (IFO 14578, FERM BP-1322)

Plasmid extraction from Escherichia coli C600 (pPA350) was performed in accordance with the method described in "Molecular Cloning", pp.86-93. That is, Escherichia coli C600 (pPA350) was inoculated to LB medium (10 g/$\ell$ Bacto-tryptone, 5 g/$\ell$ yeast extract, 5 g/$\ell$ sodium chloride), and chloramphenicol was added to 140 $\mu$g/m$\ell$ for amplifying the plasmid. After overnight cultivation, cells were collected and washed. To the washed cells were added lysozyme and then a 0.2N-sodium hydroxide solution containing 1% sodium lauryl sulfate to thereby the cells. After adding 5M-potassium acetate, the cells were centrifuged to thereby give a supernatant containing the plasmid. To the supernatant was added 0.6-fold volume of isopropanol to thereby precipitate the plasmid DNA. The precipitate, after washing with ethanol, was dissolved in TE buffer (10 mM Tris-HCl buffer, 1 mM EDTA, pH 8.0). To the resulting solution was added cesium chloride to a specific gravity of 1.60 and then ethidium bromide to a final concentration of 600 $\mu$g/m$\ell$. The mixture was subjected to 12 hours of centrifugation at 20°C and 50,000 rpm using an ultracentrifuge (roter, V$_{65}$Ti), and the plasmid band detectable by ultraviolet was taken. After extraction and removal of the ethidium bromide with n-butanol, the DNA was dialyzed to TE buffer, whereby the recombinant plasmid pPA350 in an amount of about 100 $\mu$g was obtained from 1 $\ell$ of the culture broth.

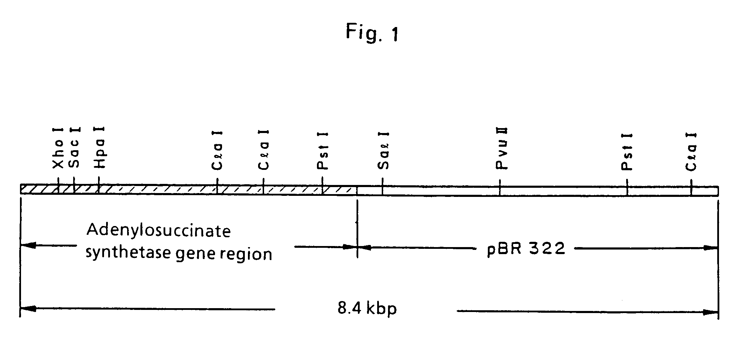

The plasmid pPA350 was digested with various restriction enzymes, and, based on the molecular weight of the Hin dIII digest of lamda phage DNA, a restriction site map as shown in Fig. 1 was derived from the agarose gel electrophoresis migration pattern.

v) Isolation of pC194 chloramphenicol acetyl-transferase gene (cat)

pC194 (3 $\mu$g) was digested with the restriction enzymes Hpa II and Sau 3AI, and the cohesive ends were converted to blunt ends by means of T4 DNA polymerase (Takara Shuzo, Japan). Separately, pUC19 (1 $\mu$g) was digested with the restriction enzyme Hinc II. Both the cleaved fragments were mixed together and ligated together by means of T4 DNA ligase. Using this reaction mixture, Escherichia coli MM294 (ATCC 33625) was transformed to thereby obtain an ampicillin-resistant and chloramphenicol-resistant transformant, namely Escherichia coli MM294 (pCAT15). From this transformant the recombinant plasmid was extracted in accordance with the method of the previous term, whereby about 2000 $\mu$g DNA was obtained from 1 $\ell$ of the culture broth, and the DNA was designated pCAT15. 30 $\mu$g of pCAT15 was double digested with the restriction enzymes Eco RI and Hin dIII, and the cohesive ends were converted to blunt ends by means of T4 DNA polymerase. Thereafter, agarose gel electrophoresis was performed, and an agarose gel fragment for the portion corresponding to the mobility of 1.1 kb DNA was cut out. Then, using a Unidirectional Electroeluter, said DNA was extracted from said agarose gel fragment, and the DNA was subjected to ethanol precipitation. The precipitate was dissolved in TE buffer

Example 2

Construction of plasmid DNA comprising adenylosuccinate synthetase gene inserted chloramphenicol acetyltransferase gene therein

After complete digestion with the restriction enzyme Hpa I, 3 $\mu$g of pPA350 was heated at 65°C for 15 minutes and subjected to ethanol precipitation, and the precipitate was dissolved in TE buffer.

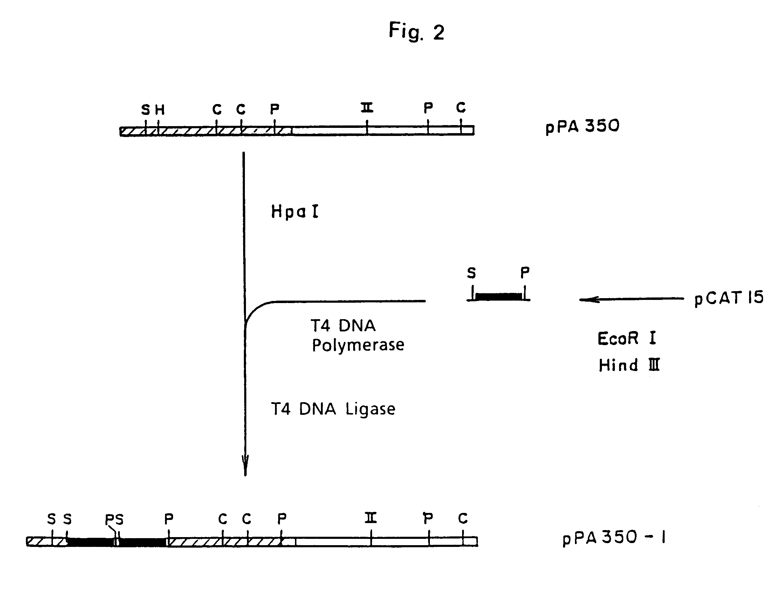

The resulting solution and the DNA fragmentcontaining solution obtained in the previous term were mixed together, and ligation reaction was conducted by means of T4 DNA ligase. Using a 5 $\mu\ell$ portion of the reaction mixture, Escherichia coli MM294 competent cells were transformed to thereby obtain an ampicillin-resistant and chloramphenicol-resistant transformant, namely Escherichia coli MM294 (pPA350-1) (IFO 14579, FERM BP-1323). Then, the plasmid was extracted from the transformant Escherichia coli MM294 (pPA350-1) in accordance with the method described above in Example 1-iv, and was designated pPA350-1. The plasmid pPA350-1 was cleaved with various restriction enzymes, and, based on the molecular weight of the Hin dIII cleavaged product of lamda phage DNA, a restriction map was derived from the agarose gel electrophoresis pattern (Fig. 2). From the restriction pattern, it is evident that said DNA is a 10.5 kb DNA comprising pBR322 and the inserted fragment, and it has the structure in which the DNA fragment containing the chloramphenicol acetyltransferase gene of pCAT15 is inserted in the Hpa I site on the adenylosuccinate synthetase gene of Bacillus subtilis. The method of constructing pPA350-1 and the restriction enzyme map are shown in Fig. 2.

Example 3

i) Construction of new Bacillus strain carrying inactivated adenylosuccinate synthetase gene DNA into which chloramphenicol acetyltransferase gene is inserted on the chromosome DNA

By the conventional method of deriving an auxotroph, a purine nucleoside phosphorylase-deficient 5′-guanylate reductasse-deficient transformant, namely Bacillus subtilis BM 1022 (IFO 14580, FERM BP-1324) was derived from Bacillus subtilis MI 114. Then, competent cells of said strain were prepared in accordance with the method of C. Anagnostpoulos and J. Spizizen. To 1 m$\ell$ of a suspension of said competent cells was added a 10.5 kb DNA fragment obtained by cleaving the plasmid obtained in Example 2 with Pvu II, and the suspension, after shaking at 37°C for 1 hour, was spread over LB agar plate containing 10 $\mu$g/m$\ell$ chloramphenicol and incubated at 37°C for one day. From the formed colony was obtained a chloramphenicol-resistant transformant, namely Bacillus subtilis BM 1051 (IFO 14581, FERM BP-1325). Said transformant was inoculated to both M-9 CAT agar plate and another above medium plate containing adenine to the above medium, and its growth was examined; as a result, it was confirmed that said chloramphenicol-resistannt transformant is adenine requirement at the same time.

ii) Back mutation test on adenine requirement of Bacillus subtilis BM 1051

Bacillus subtilis BM 1051 was inoculated to 20 m$\ell$ of the seed medium as described in Table 1, and was subjected to shaking cultivation at 37°C for 18 hours. Thereafter, 1 m$\ell$ of the culture medium was transferred to the main fermentation medium (20 m$\ell$) as described in Table 1, and was subjected to shaking cultivation at 37°C for 2 days. After repeating subinoculation 5 times as above, the viable cells in the culture broth were counted; the viable cell count was found to be 1 $\times$ 10$^9$ per m$\ell$ culture broth, all of which had adenine requirement and none of which was a revertant.

Separately, Bacillus subtilis BM 1022 was subjected to the conventional method of deriving an auxotroph. That is, said strain, after treatment with nitroso guanidine, was spread over M-9 CAT agar plate medium containing 50 mg/$\ell$ of adenine, and was incubated at 37°C for 1 day. The formed colony, by the replica plate method, was transferred to both M-9 CAT agar plate medium (the same composition as above except that adenine was not contained) and adenine-containing M-9 CAT agar plate medium, and was incubated at 37°C overnight, whereby an adenylosuccinate synthetase-deficient strain, namely Bacillus subtilis BM 1032 (IFO 14459, FERM BP-1242) was obtained from the colony, which can grow in the presence of adenine but which cannot grow in the absence of adenine.

This adenine-repuiring strain Bacillus subtilis BM 1032 was subjected to the same procedure as above in order to examine frequency of back mutation; as a result, the viable cell count per mℓ culture broth was found to be $1 \times 10^9$, 15% of which, namely $1.5 \times 10^8$ cells were revertants having no adenine requirement.

iii) Inosine and guanosine productivity of Bacillus subtilis BM 1051

Bacillus subtilis BM 1051 was inoculated to a conical fask of 1ℓ capacity containing 125 mℓ of the seed medium shown in Table 1, and was subjected to shaking culture at 37°C for 12 hours. The entire amount of the culture broth was then transferred to a jar of 5ℓ capacity containing 2.5ℓ of the main fermentation medium as described in Table 1, and was cultivated at 37°C for 72 hours while 100 mℓ of a mixed solution containing 12.5% ammonium sulfate and 6.25% urea was added at 24 hours after the inititation of the main fermentation. At the end of the cultivation, the amounts of inosine and guanosine which accumulated were examined by high performance liquid chromatography; it was confirmed that 9.4 mg/mℓ inosine and 5.0 mg/mℓ guanosine had accumulated. The transformant Bacillus subtilis BM 1032 was cultivated under the same conditions as above, leading only to the accumulation of 6.3 mg/mℓ inosine and 4.9 mg/nℓ guanosine.

## Table 1

| Composition of medium | Concentration (g/ℓ) | |
|---|---|---|
| | Seed medium | Main fermentation medium |
| Glucose | 30 | 160 |
| Sodium glutamate | 10 | 10 |
| Ammonium sulfate | | 7.5 |
| Urea | 3 | 10 |
| Corn steep liquor | 20 | 30 |
| Magnesium sulfate | 2 | 2 |
| Adenine | 0.2 | 0.075 |
| Dipotassium monohydrogenphosphate | 21 | |
| Potassium chloride | | 0.5 |
| Calcium chloride | 2 | 5 |
| Manganese sulfate | | 0.0025 |
| Calcium carbonate | | 30 |
| pH | 7.0 | 6.8 |

Example 4

In accordance with the method of Example 3, competent cells of the strain Bacillus subtilis NA-6128 (IFO 14373, FERM BP-617) were prepared, and the 8.4 kb DNA fragment obtained by cleaving pPA350 with Pvu II was added; then, the cells were shaken at 37°C for 1 hour to thereby carry out transformation. The shaken mixture was then spread over M-9 CAT agar plate medium, and, from the formed colony, an adenine requirement-repaired transformant, namely NA-6128R was obtained. Then, competent cells of said transformant were prepared, and the 10.5 kb DNA fragment obtained by cleaving and linearizing the pPA350-1 obtained in Example 2 with Pvu II was added, and this was followed by shaking at 37°C for 1 hour to thereby carry out transformation; from the colony which grew on LB medium containing 10 $\mu$g/m$\ell$ chloramphenicol, a chloramphenicol-resistant transformant, namely Bacillus subtilis NA-6201 (IFO 14582, FERM BP-1326) was derived. Based on the growability on M-9 CAT medeium and on M-9 CAT medium containing adenine, it was confirmed that said transformant has adenine-requirement.

Example 5

The strain Bacillus subtilis NA-6201 was inoculated to a conical flask of 1$\ell$ capacity containing 125 m$\ell$ of the seed medium as shown in Table 1, and was subjected to shaking cultivation at 37°C for 12 hours. The entire amount of the culture broth was transferred to a jar of 5$\ell$ capacity containing 2.5$\ell$ of the main fermentation medium of Table 1, and was cultivated at 37°C for 96 hours while 100 m$\ell$ of a mixed solution containing 12.5% ammonium sulfate and 6.25% urea was added at both 24 and 48 hours after the initiation of the main fermentation. At the end of the cultivation, the amounts of inosine and guanosine which accumulated were examined by high performance liquid chromatography; it was confirmed that 24.3 mg/m$\ell$ inosine and 18.2 mg/m$\ell$ guanosine were accumulated. The strain Bacillus subtilis NA-6128 was cultivated under the same conditions as above, leading only to the accumulation of 20.5 mg/m$\ell$ inosine and 16.1 mg/m$\ell$ guanosine.

The culture broth of the above-mentioned NA-6201 strain was examined for viable cell count and adenine-requiring revertant cell count; the viable cell count was found to be $8 \times 10^8$ per m$\ell$, none of which was an adenine-requiring revertant.

The culture broth of the above-mentioned NA-6128 strain was also examined for the presence of adenine-requiring revertants; it was confirmed that $3 \times 10^5$ revertants were present per m$\ell$ culture broth. That is, comparison of back mutation rate also evidences the effect of this invention.

Fig. 1 shows the restriction enzyme map of the recombinant plasmid containing the adenylosuccinate synthetase gene region as obtained in Example 1.

Fig. 2 shows the method of preparing the plasmid pPA350-1 obtained in Example 2. In the figure, S, H, C, P, and II respectively represent the restriction enzymes Sac I, Hpa I, Cla I, Pst I and Pvu II. The shaded regions indicate the adenylosuccinate synthetase gene region; the blacked regions indicate the chloramphenicol acetyltransferase gene region; the white regions indicate pBR322. The thin lines at both ends of the fragment cleaved from pCAT15 with Eco RI and Hin dIII indicate the DNA derived from the multicloning site of pUC19.

**Claims**

1. A DNA comprising an adenylosuccinate synthetase gene region with a DNA fragment inserted to render said gene inactivated.

2. The DNA according to claim 1, wherein said DNA fragment is a gene other than an adenylosuccinate synthetase gene.

3. The DNA according to claim 2, wherein said other gene is a selection marker gene.

4. The DNA according to claim 3, wherein the selection marker gene is a chloramphenicol acetyltransferase gene.

5. A vector with a DNA of claim 1, 2, 3 or 4 inserted therein.

6. A Bacillus subtilis strain which is a transformant resulting from transformation with the DNA as claimed in claim 1, 2, 3 or 4, which lacks adenylosuccinate synthetase and which is capable of producing inosine and/or guanosine, wherein said strain is prevented from presenting back mutation on adenine requirement, the DNA comprising an adenylossucinate synthetase gene region with a selection marker gene inserted to render said gene inactivated.

7. The Bacillus subtilis strain according to claim 6, wherein the transformant is Bacillus subtilis BM 1051 (FERM BP-1325) or Bacillus subtilis NA-6201 (FERM BP-1326).

8. A method of producing inosine and/or guanosine, which comprises cultivating in a medium the Bacillus subtilis strain of claim 6 or 7, allowing said strain to produce and accumulate inosine and/or guanosine, and harvesting inosine and/or guanosine so produced in the culture medium.

**Patentansprüche**

1. DNA, umfassend eine Adenylsuccinatsynthetase Genregion mit einem DNA-Fragment, das inseriert wurde, um das Gen zu inaktivieren.

2. DNA nach Anspruch 1, worin das DNA-Fragment ein anderes Gen ist, als ein Adenylsuccinatsynthetase Gen.

3. DNA nach Anspruch 2, worin das andere Gen ein Selektionsmarkergen ist.

4. DNA nach Anspruch 3, worin das Selektionsmarkergen ein Chloramphenicol-Acetyltransferasegen ist.

5. Vektor mit einer DNA nach Anspruch 1, 2, 3 oder 4, die dahinein inseriert ist.

6. Bacillus subtilis Stamm, der ein Tranformant ist, entstanden durch Transformation mit der DNA nach Anspruch 1, 2, 3 oder 4, dem Adenylsuccinatsynthetase fehlt und der befähigt ist, Inosin und/oder Guanosin zu bilden, wobei der Stamm an der Ausbildung einer Rückmutation auf Adeninbedarf gehindert ist, wobei die DNA eine Adenylsuccinatsynthetase Genregion mit einem Selektionsmarkergen, das zur Inaktivierung des Gens inseriert ist, umfaßt.

7. Bacillus subtilis Stamm gemäß Anspruch 6, worin der Transformant Bacillus subtilis BM 1051 (FERM BP-1325) oder Bacillus subtilis NA-6201 (FERM BP-1326) ist.

8. Verfahren zur Herstellung von Inosin und/oder Guanosin, umfassend das Kultivieren des Bacillus subtilis Stammes nach Anspruch 6 oder 7 in einem Medium, wobei man den Stamm Inosin und/oder Guanosin bilden und akkumulieren läßt und Ernten des auf diese Weise in dem Kulturmedium gebildeten Inosins und/oder Guanosins.

**Revendications**

1. ADN comprenant une région de gène adénylosuccinate synthétase avec un fragment de ADN inséré pour rendre ledit gène inactif.

2. ADN selon la revendication 1, où ledit fragment ADN est un gène autre que le gène adénylosuccinate synthétase.

3. ADN selon la revendication 2, où ledit autre gène est un gène marqueur de sélection.

4. ADN selon la revendication 3, où le gène marqueur de sélection est un gène chloramphénicol acétyltransférase.

5. Vecteur avec un ADN selon la revendication 1, 2, 3 ou 4 qui y est inséré.

6. Une souche Bacillus subtilis qui est un transformant résultant de la transformation avec l'ADN tel que revendiqué dans la revendication 1,2,3 ou 4, qui est dénué d'adénylosuccinate synthetase et qui est capable de produire l'inosine et/ou de la guanosine, où ladite souche est empêchée de présenter une mutation reverse concernant le besoin en adénine, l'ADN comprenant une région de gène adénylosuccinate synthétase avec un gène marqueur de sélection insérée pour rendre ledit gène inactivé.

**7.** Souche Bacillus subtilis selon la revendication 6, où le transformant est Bacillus Subtilis BM-1051/FERM BP-1325) ou Bacillus subtilis NA-6201/FERM BP-1326).

**8.** Procédé de production d'inosine et/ou de guanosine, qui consiste à cultiver la souche Bacillus subtilis selon la revendication 6 ou 7, dans un milieu, à laisser cette souche à produire et à accumuler l'inosine et/ou la guanosine, et à récolter l'inosine et/ou la guanosine ainsi produite dans le milieu de culture.

Fig. 1

Fig. 2